# EUROPÄISCHE PATENTSCHRIFT

(11) **EP 0 708 815 B1**
(45) Veröffentlichungstag und Bekanntmachung des Hinweises auf die Patenterteilung: **24.02.1999**
(21) Anmeldenummer: 94924231.7
(22) Anmeldetag: 04.07.1994
(51) Int. Cl.: C11D 1/52, C11D 1/835

(54) **VERWENDUNG VON FETTSÄURE-N-ALKYLPOLYHYDROXYALKYLAMIDEN ZUR HERSTELLUNG VON KLARSPÜLMITTELN FÜR DIE MASCHINELLE REINIGUNG HARTER OBERFLÄCHEN**
USE OF FATTY ACID N-ALYKL POLYHYDROXYALKYLAMIDES IN THE PROCESSING OF CLEAR-RINSING AGENTS FOR THE MACHINE-CLEANING OF HARD SURFACES
UTILISATION DE N-ALKYLPOLYHYDROXYAMIDES D'ACIDES GRAS DANS LA FORMULATION DE LIQUIDES DE RINCAGE POUR LE NETTOYAGE MECANIQUE DE SURFACES DURES

(30) Priorität: 12.07.1993 DE 4323253
(43) Veröffentlichungstag der Anmeldung: 01.05.1996
(73) Patentinhaber: Henkel Kommanditgesellschaft auf Aktien, 40191 Düsseldorf (DE)
(72) Erfinder: FABRY, Bernd, D-41352 Korschenbroich (DE); HÄRER, Jürgen, D-40597 Düsseldorf (DE); BURG, Birgit, D-46519 Alpen (DE); NEJTEK, Marica, D-40789 Monheim (DE); JESCHKE, Peter, D-41468 Neuss (DE); HEES, Udo, D-56727 Mayen (DE)
(86) Internationale Anmeldenummer: EP9402187
(87) Internationale Veröffentlichungsnummer: WO9502666

(56) Entgegenhaltungen:
- EP-A- 0 054 895
- EP-A- 0 095 136
- EP-A- 0 285 768
- WO-A-92/06156
- WO-A-92/06161
- WO-A-92/22629
- SEIFEN, OLE, FETTE, WACHSE, Bd.119, Nr.13, 7. Oktober 1993, AUGSBURG DE Seiten 794 - 808, XP398733 M.STALMANS ET AL. 'The Environmental Properties of Glucose Amide- a New Nonionic Surfactant'

## Beschreibung

### Gebiet der Erfindung

Die Erfindung betrifft die Verwendung von Fettsäure-N-alkylpolyhydroxyalkylglucamiden zur Herstellung neuer Klarspülmittel für die maschinelle Reinigung harter Oberflächen.

### Stand der Technik

Marktübliche Klarspülmittel stellen Gemische aus schwachschäumenden Fettalkoholpolyethylen/polypropylenglycolethern, Lösungsvermittlern (z. B. Cumolsulfonat), organischen Säuren (z.B. Citronensäure) und Lösungsmitteln (z.B. Ethanol) dar. Die Aufgabe dieser Mittel besteht darin, die Grenzflächenspannung des Wassers so zu beeinflussen, daß es in einem möglichst dünnen, zusammenhängenden Film vom Spülgut ablaufen kann, so daß beim anschließenden Trocknungsvorgang keine Wassertropfen, Streifen oder Filme zurückbleiben. Eine Übersicht über die Zusammensetzung von Klarspülern und Methoden zur Leistungsüberprüfung findet sich von W.Schirmer et al. in **Tens. Surf. Det.** **28,** **313 (1991)**.

Bei der Verwendung moderner phosphatfreier und niederalkalischer Reiniger für das maschinelle Geschirrspülen kann es ferner zur Bildung von Kalk- bzw. Silicatbelägen auf dem Spülgut und im Maschineninnenraum kommen kann, da das Calciumbindevermögen dieser Reiniger geringer ist, als das der klassischen phosphathaltigen Produkte. Störende Kalk- bzw. Silicatbeläge treten insbesondere dann auf, wenn das Spülwasser der Geschirrspülmaschine nicht oder nicht ausreichend enthärtet wird und eine Wasserhärte von 4°d überschritten wird. In solchen Fällen lassen sich Kalk- bzw. Silicatbeläge wirksam vermeiden, wenn über den Klarspüler Citronensäure in den Klarspülgang dosiert wird. Da jedoch die normalerweise während des Klarspülgangs zugegebenen Klarspülermengen mit 3 ml - 6 ml sehr gering sind, muß zur Erreichung einer ausreichenden Säure- bzw. Komplexierkapazität der Citronensäuregehalt in Klarspülerformulierungen, die eine effektive Belagsinhibierung gewährleisten sollen, relativ hoch sein. Solche hohen Citronensäuregehalte unterstützen die Wirkung der Phosphatersatzstoffe und gewährleisten fleckenloses Geschirr.

In der Internationalen Anmeldung **WO 88/09369** werden flüssige wäßrige Mittel für das maschinelle Reinigen von Geschirr beschrieben, die C₈₋₁₆-Alkyloligoglucoside, schwach schäumende Fettalkoholpolyglycolether und Alkalicitrate enthalten. Derartige Reinigungsmittel sind jedoch als Klarspüler ungeeignet.

In der Europäischen Patentanmeldung **EP-A2 0 432 836** (Unilever) werden Klarspülerformulierungen für das maschinelle Geschirrspülen beschrieben, die nur ein Tensid und zwar ausschließlich Alkyloligoglucoside sowie als weitere Bestandteile Entschäumer und Verdickungsmittel enthalten. Entschäumer sind notwendiger Bestandteil dieser Rezepturen, da Alkyloligoglucoside im allgemeinen starke Schäumer sind und in den beschriebenen Klarspülerformulierungen bei Anwendung in der Spülmaschine eine nicht akzeptable Schaumbelastung verursachen würden. Entschäumer sind aber nur dann wirksam, wenn sie sich in dem zu entschäumenden Medium nicht lösen. Daher sind auch die Alkyloligoglucosidlösungen und die Entschäumer in den aufgeführten Klarspüler-Beispielen nicht miteinander mischbar. Es würden also zwei Phasen existieren, wenn nicht zu deren Dispergierung ein Verdickungsmittel verwendet worden wäre, das für eine gewisse Homogenisierung sorgt. Ein Nachteil einer solchen Formulierung bleibt aber einerseits die durch den Entschäumer verursachte latente Inhomogenität. Entschäumer und Tensidlösung des Klarspülers trennen sich trotz des Gehaltes an Verdickungsmittel bereits nach kurzer Lagerzeit. Ein solches Produkt ist verständlicherweise für Kunden von Markenartikeln und im Großverbrauch gleichermaßen ungeeignet, denn hier werden Produkte gefordert, die über einen längeren Zeitraum, mindestens jedoch ein Jahr lang, lagerstabil sein müssen. Es kommen also nur einphasige Formulierungen in Betracht, in denen alle Rezepturbestandteile homogen gelöst sind und die nicht nur lagerstabil bleiben, sondern sich außerdem auch während des Auf- und Abkühlvorgangs des Spülcyclus nicht trennen, d.h., es muß eine Phasenstabilität im Temperaturbereich der Geschirrspülmaschine von 0 °C - 65 °C gewährleistet sein. Andererseits sind die bekannten Produkte so lange sie noch homogen sind, so zähflüssig, daß sie sich nicht über die Klarspülerdosiervorrichtung einer Haushaltsgeschirrspülmaschine eingießen lassen.

In der Geschirrspülmaschine wird eine Vielzahl unterschiedlicher Materialien (Glas, Metall, Silber, Kunststoff, Porzellan) gereinigt. Diese Materialvielfalt muß im Klarspülgang möglichst gut benetzt werden. Klarspülerformulierungen, die als Tensidkomponente ausschließlich Alkylpolyglykoside enthalten, erfüllen diese Anforderungen nicht oder nur in geringem Umfang, so daß der Klarspül- bzw. Trocknungseffekt insbesondere bei Kunststoffoberflächen nicht zufriedenstellend ist.

Für den Einsatz in Reinigungsmitteln, also auch in Klarspülerformulierungen, kommen ferner heute nur noch Rezepturbestandteile in Frage, die biologisch vollständig abbaubar und toxikologisch unbedenklich sind. Ein besonderes Interesse kommt dabei lösungsmittelfreien Produkten zu.

Die Aufgabe der vorliegenden Erfindung hat somit darin bestanden, neue ökologisch und toxikologisch einwandfreie Formulierungen bereitzustellen, die bezüglich der anwendungstechnischen Eigenschaften gleiche Resultate liefern, wie marktgängige Klarspüler und die vorstehend genannten Nachteile nicht aufweisen.

### Beschreibung der Erfindung

Gegenstand der Erfindung ist die Herstellung von Klarspülmitteln für die maschinelle Reinigung harter Oberflächen unter Verwendung von Fettsäure-N-alkylpolyhydroxyalkylamiden der Formel (I), 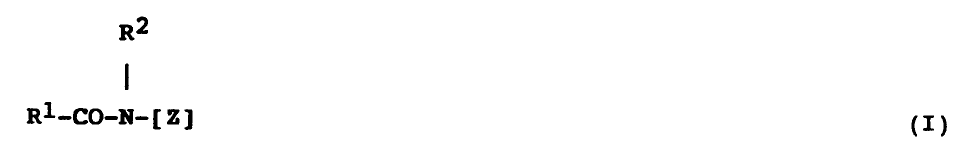 in der R¹CO für einen aliphatischen Acylrest mit 6 bis 22 Kohlenstoffatomen, R² für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht, sowie gegebenenfalls weitere nichtionische Tenside und übliche Hilfs- und Zusatzstoffe.

Überraschenderweise wurde gefunden, daß Klarspüler mit einem Gehalt an Fettsäure-N-alkylpolyhydroxyalkylamiden und insbesondere Fettsäure-N-methylglucamiden nicht nur eine hohe ökotoxikologische Verträglichkeit aufweisen, sondern die Anforderungen an ein Markenprodukt auch im Hinblick auf die anwendungstechnischen Eigenschaften voll erfüllen.

Ein weiterer Vorteil dieser Klarspüler ist, daß zur Herstellung ihrer homogenen, niedrigviskosen und daher leicht zu dosierenden Lösungen keine weiteren, meist inerten und daher für die Trocknung und den Klarspüleffekt uneffektiven Lösungsvermittler wie z. B. Natriumcumolsulfonat, Ethanol oder Glucosesirup benötigt werden, es sei denn, sie werden in geringen Mengen für die Einarbeitung von Farb- und/oder Duftstoffen erforderlich.

Als besonders vorteilhaft haben sich schließlich binäre und ternäre Kombinationen der Fettsäure-N-alkylpolyhydroxyalkylamide mit Fettalkoholpolyglycolethern, Mischethern und/oder Alkyloligoglucosiden erwiesen. Derartige Formulierungen sind extrem schaumarm und benetzen auch unterschiedliche Materialien in gleicher Weise gut. Ein besonderer Klarspüleffekt wird an Kunststoffoberflächen erzielt.

### Fettsäure-N-alkylpolyhydroxyalkylamide

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Eine Übersicht hierzu findet sich von von H.Kelkenberg in Tens.Surf.Det. **25**, 8 (**1988**). Hinsichtlich der Verfahren zu ihrer Herstellung sei auf die US-Patentschriften **US 1 985 424, US 2 016 962** und **US 2 703 798**, die Deutsche Auslegeschrift **DE-AS 12 61 861** sowie die Internationale Patentanmeldung **WO 92/06984** verwiesen.

Auch die Verwendung der genannten Fettsäureamide ist Gegenstand einer Vielzahl von Veröffentlichungen. Aus der Europäischen Patentanmeldung **EP-Al 0 285 768** (Hüls) ist beispielsweise ihr Einsatz als Verdickungsmittel bekannt. In der Französischen Offenlegungsschrift **FR-A 1 580 491** (Henkel) werden wäßrige Detergensgemische auf Basis von Sulfaten und/oder Sulfonaten, Niotensiden und gegebenfalls Seifen beschrieben, die Fettsäure-N-alkylglucamide als Schaumregulatoren enthalten.

Gegenstand der Internationalen Patentanmeldungen **WO 92/6153; 6156; 6157; 6158; 6159** und **6160** (Procter & Gamble) sind Mischungen von Fettsäure-N-alkylglucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/6152; 6154; 6155; 6161; 6162; 6164; 6170; 6171** und **6172** (Procter & Gamble) beschrieben. Einen Hinweis auf die vorteilhafte Verwendung dieser Stoffe in Klarspülern enthalten die Schriften jedoch nicht.

Vorzugsweise leiten sich die in den Mitteln einzusetzenden Fettsäureamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel (II) wiedergegeben werden: 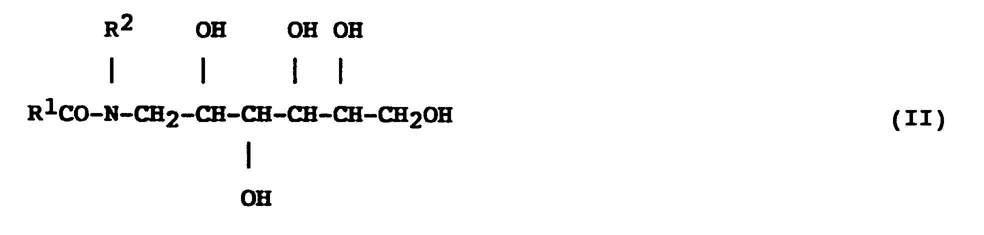 Vorzugsweise kommen als Rohstoffe für die erfindungsgemäße Herstellung der Klarspüler Fettsäure-N-alkylpolyhydroxyalkylamide der Formel (I) in Betracht, in der R¹CO für einen Acylrest mit 6 bis 12 Kohlenstoffatomen, R² für eine Methylgruppe und [Z] für reduzierten Glucoserest steht. Dementsprechend sind Fettsäure-N-methylglucamide besonders bevorzugt, die durch reduktive Aminierung von Glucose mit Methylamin und anschließende Acylierung mit einer C₆-C₁₂-Fettsäure bzw. einem entsprechenden Derivat erhalten werden. Bei der Auswahl der Fettsäure kommt insbesondere eine C₈-C₁₀-Fraktion in Frage, die beispielsweise als Vorlauf bei der Destillation von technischer Kokosfettsäure anfällt.

### Nichtionische Tenside

Nichtionische Tenside stellen zwar eine fakultative, jedoch bevorzugte Komponente der Klarspülmittel dar. Grundsätzlich kommen für diesen Zweck alle nichtionischen Tenside, insbesondere jedoch Fettalkoholpolyethylenglycolether, Fettalkoholpolyethylen/polypropylenglycolether, Mischether und/oder Alkyloligoglucoside in Betracht.

**Fettalkoholpolyethylenglycolether**, die in Betracht kommen, folgen der Formel (III),

R³O-(CH₂CH₂O)ₙ₁H (III)

in der R³ für einen linearen oder verzweigten Alkyl- und/oder Alkenylrest mit 6 bis 22, vorzugsweise 12 bis 18 Kohlenstoffatomen und nl für Zahlen von 1 bis 5 steht.

Die genannten Stoffe stellen bekannte Handelsprodukte dar. Typische Beispiele sind Anlagerungsprodukte von durchschnittlich 2 bzw. 4 Mol Ethylenoxid an technischen C_{12/14}-Kokosfettalkohol (Dehydol^{(R)} LS-2 bzw. LS-4, Fa.Henkel KGaA) oder Anlagerungsprodukte von durchschnittlich 4 Mol Ethylenoxid an C_{14/15}-Oxoalkohole (Dobanol^{(R)} 45-4, Fa.Shell). Die Produkte können eine konventionelle oder auch eingeengte Homologenverteilung aufweisen.

Unter **Fettalkoholpolyethylen/polypropylenglycolethern** sind nichtionische Tenside der Formel (**IV**) zu verstehen, 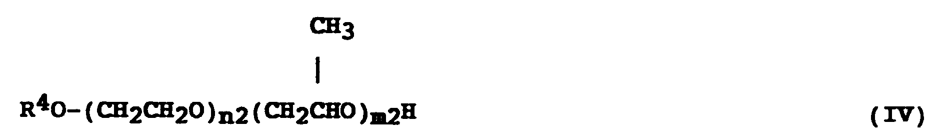 in der R⁴ für einen linearen oder verzweigten Alkyl- und/oder Alkenylrest mit 6 bis 22, vorzugsweise 12 bis 18 Kohlenstoffatomen, n2 für Zahlen von 1 bis 5 und m2 für Zahlen von 1 bis 4 steht.

Auch diese Stoffe stellen bekannte Handelsprodukte dar. Ein typisches Beispiel ist ein Anlagerungsprodukt von durchschnittlich 5 Mol Ethylenoxid und 4 Mol Propylenoxid an technischen C_{12/14}-Kokosfettalkohol (Dehydol^{(R)} LS-54, Fa.Henkel KGaA)

Unter **Mischethern** sind endgruppenverschlossene Fettalkoholpolyglycolether zu verstehen, die der Formel (**IV**) folgen, 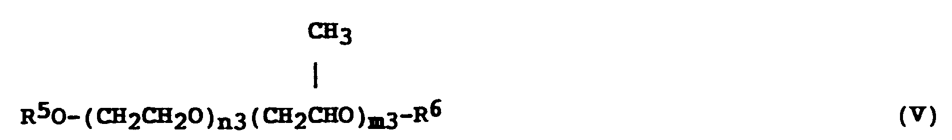 in der R⁵ für einen linearen oder verzweigten Alkyl- und/oder Alkenylrest mit 6 bis 22, vorzugsweise 12 bis 18 Kohlenstoffatomen, n3 für Zahlen von 1 bis 10, m2 für 0 oder Zahlen von 1 bis 4 und R⁶ für einen Alkylrest mit 1 bis 4 Kohlenstoffatomen oder einen Benzylrest steht.

Typische Beispiele sind Mischether der Formel (**V**), in der R⁵ für einen technischen C_{12/14}-Kokosalkylrest, n3 für 5 bzw. 10, m3 für 0 und R⁶ für eine Butylgruppe steht (Dehypon^{(R)} LS-54 bzw. LS-104, Fa. Henkel KGaA). Die Verwendung von butyl- bzw. benzylgruppenverschlossenen Mischethern ist aus anwendungstechnischen Gründen besonders bevorzugt.

**Alkyloligoglucoside** der Formel (**VI**)

R⁷O-[G]ₚ (VI)

in der R⁷ für einen Alkylrest mit 6 bis 12 Kohlenstoffatomen, G für einen Glucoserest und p für Zahlen von 1 bis 10 steht, stellen bekannte Stoffe dar, die nach den einschlägigen Verfahren der präparativen organischen Chemie erhalten werden können. Stellvertretend für das umfangreiche Schrifttum sei hier auf die Schriften **EP-Al-0 301 298** und **WO 90/3977** verwiesen.

Der Alkylrest R⁷ kann sich von primären Alkoholen mit 6 bis 12, vorzugsweise 8 bis 10 Kohlenstoffatomen ableiten.

Typische Beispiele sind Capronalkohol, Caprylalkohol, Caprinalkohol, Undecylalkohol und Laurylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Die Indexzahl p in der allgemeinen Formel (**VI**) gibt den Oligomerisierungsgrad (DP-Grad), d. h. die Verteilung von Mono- und Oligoglucosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglucosid eine analytisch ermittelte rechnerische Größe, die meistens eine gebrochene Zahl darstellt. Vorzugsweise werden Alkyloligoglucoside mit einem mittleren Oligomerisierungsgrad p von 1,1 bis 3,0 eingesetzt. Aus anwendungstechnischer Sicht sind solche Alkyloligoglucoside bevorzugt, deren Oligomerisierungsgrad kleiner als 1,7 ist und insbesondere zwischen 1,2 und 1,4 liegt.

### Hilfs- und Zusatzstoffe

Als wichtigste Zusatzstoffe kommen ein- und mehrwertige Carbonsäuren, vorzugsweise Hydroxycarbonsäuren in Betracht. Typische Beispiele sind Äpfelsäure (Monohydroxybernsteinsäure), Weinsäure (Dihydroxybernsteinsäure); gesättigte aliphatische Dicarbonsäuren wie Oxalsäure, Malonsäure, Bernsteinsäure, Glutarsäure, Adipinsäure; Gluconsäure (Hexan-Pentahydroxy-1-Carbonsäure), vorzugsweise jedoch wasserfreie Citronensäure. Sie können in Mengen von etwa 1 bis 50, vorzugsweise von etwa 1 bis 30 Gew.-% eingesetzt werden.

Als weitere Zusatzstoffe kommen vor allem Farb- und Duftstoffe in Frage.

### Klarspüler-Formulierungen

Typische Formulierungen der Klarspüler können beispielsweise folgende Zusammensetzung aufweisen (ad 100 Gew.-% Wasser):

| | | |
|---|---|---|
| 0,5 | bis 20 Gew.-% | Fettsäure-N-alkylpolyhydroxyalkylamide, |
| 0 | bis 20 Gew.-% | Alkyloligoglucoside, |
| 0 | bis 20 Gew.-% | Fettalkoholpolyglycolether, |
| 0 | bis 20 Gew.-% | Mischether, |
| 1 | bis 50 Gew.-% | Carbonsäure. |

Besonders vorteilhaft sind Rezepturen enthaltend

| | | |
|---|---|---|
| 1 | bis 10 Gew.-% | Fettsäure-N-alkylglucamide, |
| 3 | bis 10 Gew.-% | Alkyloligoglucoside, |
| 3 | bis 10 Gew.-% | Fettalkoholpolyglycolether und/oder Mischether, |
| 1 | bis 30 Gew.-% | Citronensäure. |

### Gewerbliche Anwendbarkeit

Die Klarspülmittel enthalten ökotoxikologisch besonders verträgliche Inhaltsstoffe, lassen sich lösungsmittelfrei formulieren und zeigen ein ausgezeichnetes Netzvermögen gegenüber unterschiedlichsten Materialien.

Ein weiterer Gegenstand der Erfindung betrifft daher die Verwendung von Fettsäure-N-alkylpolyhydroxyalkylamiden, insbesondere Fettsäure-N-alkylglucamiden zur Herstellung von Klarspülmitteln für die maschinelle Reinigung harter Oberflächen, insbesondere Geschirr, in denen sie in Mengen von 0,5 bis 20, vorzugsweise 1 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiele

### I. Eingesetzte Tenside

Al) Octansäure-N-methylglucamid
A2) Decansäure-N-methylglucamid
A3) C₆₋₁₂-Fettsäure-N-methylglucamid
B1) C₈₋₁₀-Alkyloligoglucosid (DP = 1,6) Plantaren^{(R)} APG 225, Fa.Henkel KGaA, Düsseldorf/FRG
C1) C_{12/14}-Kokosfettalkohol-4EO-Addukt Dehydol^{(R)} LS-4, Fa.Henkel KGaA, Düsseldorf/FRG
C2) C_{12/14}-Kokosfettalkohol-5EO-4PO-Addukt Dehydol^{(R)} LS-54, Fa.Henkel KGaA, Düsseldorf/FRG
C3) C_{12/14}-Kokosfettalkohol-10 EO-butylether Dehypon^{(R)} LS-104, Fa.Henkel KGaA, Düsseldorf/FRG

### II. Anwendungstechnische Prüfung der Klarspüler

a) Schaumverhalten:
   Die Schaumentwicklung des Klarspülers wurde mit Hilfe eines Umwälzdruck-Meßgeräts ermittelt. Der Klarspüler (3 ml) wurde hierbei im Klarspülgang bei 50 °C von Hand dosiert. Dabei bedeuten:
   0 Punkte = keine Schaumentwicklung
   1 Punkt = schwache Schaumentwicklung
   2 Punkte = mittlere Schaumentwicklung (noch akzeptabel)
   3 Punkte = starke Schaumentwicklung
b) Trocknung:
   15 Minuten nach Beendigung des Spülprogramms wurde die Tür der Geschirrspülmaschine vollständig geöffnet. Nach 5 Minuten wurde die Trocknung durch Auszählen der Resttropfen auf den unten aufgeführten Geschirrteilen bestimmt. Bewertung:
   0 Punkte = mehr als 5 Tropfen
   1 Punkt = 5 Tropfen
   2 Punkte = 4 Tropfen
   3 Punkte = 3 Tropfen
   4 Punkte = 2 Tropfen
   5 Punkte = 1 Tropfen
   6 Punkte = 0 Tropfen (optimale Trocknung)
c) Klarspüleffekt:
   Nach Beurteilung der Trocknung wurden die Geschirrteile außerhalb der Geschirrspülmaschine 30 Minuten zum Abkühlen abgestellt und dann unter Beleuchtung in einem schwarzen Kasten visuell abgemustert. Beurteilt wurden die auf dem Geschirr und Besteck verbliebenen eingetrockneten Resttropfen, Schlieren, Beläge, trüben Filme usw. Bewertung:
   0 Punkte = schlechter Klarspüleffekt
   8 Punkte = optimaler Klarspüleffekt
d) Für die Leistungsprüfungen b) und c) wurden die Versuche in der Geschirrspülmaschine Bauknecht GSF 1162 mit enthärtetem Wasser durchgeführt. Dazu wurde das 65°C Normalprogramm gewählt. Im Reinigungsgang wurden 40 ml Somat^{(R)} Reiniger (Henkel) dosiert. Die Klarspülermenge betrug 3 ml und wurde von Hand bei 50°C im Klarspülgang dosiert. Die Salzbelastung des Wassers lag zwischen 600 und 700 mg/l. Pro Klarspülerrezeptur wurden 3 Spülgänge durchgeführt. Zur Beurteilung der Trocknung sowie des Klarspüleffekts wurden folgende Geschirrteile eingesetzt:
   o Gläser "Neckar-Becher" (Fa. Schott-Zwiesel), 6 Stück
   o Edelstahlmesser "Brasilia" (Fa. WMF), 3 Stück
   o weiße Prozellan-Eßteller (Fa. Arzberg), 3 Stück
   o rote Kunststoffteller "Valon-Eßteller" (Fa. Haßmann), 3 Stück

### Beispiele 1 bis 3:

**Tab.1:**

| Klarspüler mit Fettsäure-N-methylglucamiden Prozentangaben als Gew.-% ad 100 Gew.-% Wasser | | | | | | | |
|---|---|---|---|---|---|---|---|
| Bsp. | A | c(A) % | CS % | DS % | St. °C | Aus. | S |
| 1 | A1 | 15,0 | 3,0 | 0,5 | >70 | klar | 0 |
| 2 | A2 | 15,0 | 3,0 | 0,5 | >70 | klar | 0 |
| 3 | A3 | 15,0 | 3,0 | 0,5 | >70 | klar | 1 |
| Legende: c(A) : Konzentration A CS : Citronensäure, wasserfrei DS : Duftstoff St. : Stabilität Aus. : Aussehen der Lösung S. : Schaumentwicklung | | | | | | | |

### Beispiele 4 bis 6:

**Tab.2:**

| Klarspüler mit Fettsäure-N-methylglucamiden und Fettalkoholpolyethylenglycolethern Prozentangaben als Gew.-% ad 100 Gew.-% Wasser | | | | | | | | |
|---|---|---|---|---|---|---|---|---|
| Bsp. | Glu | c(Glu) % | c(C1) % | CS % | DS °C | St. | Aus. | S |
| 4 | A1 | 9,0 | 6,0 | 3,0 | 0,5 | >70 | klar | 0 |
| 5 | A2 | 9,0 | 6,0 | 3,0 | 0,5 | >70 | klar | 1 |
| 6 | A3 | 9,0 | 6,0 | 3,0 | 0,5 | >70 | klar | 1 |

### Beispiele 7 bis 9:

**Tab.3:**

| Klarspüler mit Fettsäure-N-methylglucamiden und Fettalkohol-EO/PO-Addukten Prozentangaben als Gew.-% ad 100 Gew.-% Wasser | | | | | | | | |
|---|---|---|---|---|---|---|---|---|
| Bsp. | Glu | c(Glu) % | c(C2) % | CS % | DS °C | St. | Aus. | S |
| 7 | A1 | 9,0 | 6,0 | 3,0 | 0,5 | 65 | klar | 0 |
| 8 | A2 | 9,0 | 6,0 | 3,0 | 0,5 | 65 | klar | 0 |
| 9 | A3 | 9,0 | 6,0 | 3,0 | 0,5 | 65 | klar | 0 |

### Beispiele 10 bis 12:

**Tab.4:**

| Klarspüler mit Fettsäure-N-methylglucamiden und Mischethern Prozentangaben als Gew.-% ad 100 Gew.-% Wasser | | | | | | | | |
|---|---|---|---|---|---|---|---|---|
| Bsp. | Glu | c(Glu) % | c(C3) % | CS % | DS °C | St. | Aus. | S |
| 10 | A1 | 9,0 | 6,0 | 3,0 | 0,5 | 65 | klar | 0 |
| 11 | A2 | 9,0 | 6,0 | 3,0 | 0,5 | 65 | klar | 0 |
| 12 | A3 | 9,0 | 6,0 | 3,0 | 0,5 | 65 | klar | 0 |

### Beispiele 13 bis 16:

**Tab.5:**

| Klarspüler mit Fettsäure-N-methylglucamiden, Alkyl-oligoglucosiden u. Fettalkoholpolyethylenglycolethern Prozentangaben als Gew.-% ad 100 Gew.-% Wasser | | | | | | | | |
|---|---|---|---|---|---|---|---|---|
| Bsp. | c(A1) % | c(B1) % | c(C1) % | CS % | DS °C | St. | Aus. | S |
| 13 | 7,5 | 10,7 | 0 | 3,0 | 0,5 | >70 | klar | 0 |
| 14 | 6,0 | 8,6 | 3,0 | 3,0 | 0,5 | >70 | klar | 1 |
| 15 | 4,5 | 6,4 | 3,0 | 3,0 | 0,5 | >70 | klar | 0 |
| 16 | 4,0 | 4,3 | 3,0 | 3,0 | 0,5 | 60 | klar | 1 |

**Tab.6:**

| Trocknung der Geschirrteile/Klarspüleffekt | | | | | | | | |
|---|---|---|---|---|---|---|---|---|
| Rezeptur gem.Bsp. | Gläser | | Messer | | Porzellan | | Kunstst. | |
| | T | KSE | T | KSE | T | KSE | T | KSE |
| 1 | 2,5 | 6,2 | 4,2 | 2,1 | 5,0 | 6,4 | 4,0 | 5,3 |
| 3 | 2,9 | 6,0 | 4,2 | 3,1 | 5,0 | 6,7 | 4,2 | 5,6 |
| 4 | 4,4 | 5,8 | 5,0 | 4,6 | 5,0 | 7,3 | 5,0 | 6,7 |
| 5 | 4,6 | 6,2 | 4,7 | 4,9 | 5,0 | 6,7 | 5,0 | 6,2 |
| 6 | 4,2 | 6,0 | 4,9 | 4,4 | 5,0 | 6,7 | 5,0 | 6,5 |
| 7 | 4,1 | 6,0 | 4,9 | 6,5 | 5,1 | 7,7 | 5,0 | 6,8 |
| 8 | 4,3 | 6,3 | 5,0 | 6,1 | 5,0 | 8,0 | 5,0 | 7,0 |
| 9 | 4,6 | 5,6 | 4,6 | 4,9 | 5,0 | 7,3 | 5,0 | 6,8 |
| 10 | 4,0 | 6,1 | 4,5 | 6,3 | 5,1 | 8,0 | 5,0 | 7,0 |
| 11 | 4,7 | 6,0 | 4,4 | 6,0 | 5,0 | 8,0 | 5,0 | 7,0 |
| 12 | 4,8 | 5,6 | 4,6 | 5,2 | 5,0 | 7,7 | 5,0 | 7,0 |
| 13 | 2,5 | 6,1 | 4,2 | 4,3 | 5,0 | 7,6 | 3,6 | 5,8 |
| 14 | 4,0 | 5,9 | 4,6 | 4,7 | 5,0 | 7,9 | 4,3 | 5,9 |
| 15 | 4,5 | 6,1 | 4,8 | 5,8 | 5,0 | 7,7 | 5,0 | 6,4 |
| 16 | 4,4 | 5,9 | 5,0 | 6,4 | 5,0 | 8,0 | 5,0 | 7,0 |
| V1* | 4,8 | 6,0 | 4,8 | 6,6 | 5,0 | 8,0 | 5,0 | 6,8 |
| Legende: T = Trocknung KSP = Klarspüleffekt V1 = Marktgängiger Klarspüler | | | | | | | | |

## Patentansprüche

1. Verwendung von Fettsäure-N-alkylpolyhydroxyalkylamiden der Formel (**I**), 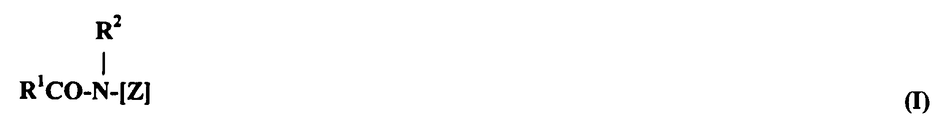 in der R¹CO flir einen aliphatischen Acylrest mit 6 bis 22 Kohlenstoffatomen, R² flir Wasserstoff, einen Alkyl- oder Hydroxyalkylrest mit 1 bis 4 Kohlenstoffatomen und [Z] flir einen linearen oder verzweigten Polyhydroxyalkylrest mit 3 bis 10 Kohlenstoffatomen und 3 bis 10 Hydroxylgruppen steht, gegebenenfalls in Abmischung mit weiteren nichtionischen Tensiden und üblichen Hilfs- und Zusatzstoffen zur Herstellung von Klarspülmitteln für die maschinelle Reinigung harter Oberflächen.

2. Verwendung nach Anspruch 1, dadurch gekennzeichnet, daß die Fettsäure-N-alkylpolyhydroxyalkylamide der Formel (I) folgen, in der R¹CO für einen Acylrest mit 6 bis 12 Kohlenstoffatomen, R² flir eine Methylgruppe und [Z] für einen reduzierten Glucoserest steht.

3. Verwendung nach den Ansprüchen 1 und 2, dadurch gekennzeichnet, daß die nichtionischen Tenside ausgewählt sind aus der Gruppe, die von Fettalkoholpolyethylenglycolethern, Fettalkoholpolyethylen/polypropylenglycolethern, Mischethern und Alkyloligoglucosiden gebildet wird.

## Claims

1. The use of fatty acid-N-alkyl polyhydroxyalkyl amides corresponding to formula (I): 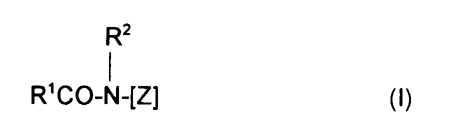 in which R¹CO is an aliphatic acyl radical containing 6 to 22 carbon atoms, R² is hydrogen, an alkyl or hydroxyalkyl radical containing 1 to 4 carbon atoms and [Z] is a linear or branched polyhydroxyalkyl radical containing 3 to 10 carbon atoms and 3 to 10 hydroxyl groups,
optionally in admixture with other nonionic surfactants and typical auxiliaries and additives, for the production of rinse aids for the machine washing of hard surfaces.

2. The use claimed in claim 1, characterized in that the fatty acid N-alkyl polyhydroxyalkyl amides correspond to formula (I), in which R¹CO is a C₆₋₁₂ acyl radical, R² is a methyl group and [Z] is a reduced glucose unit.

3. The use claimed in claims 1 and 2, characterized in that the nonionic surfactants are selected from the group consisting of fatty alcohol polyethylene glycol ethers, fatty alcohol polyethylene/polypropylene glycol ethers, mixed ethers and alkyl oligoglucosides.

## Revendications

1. Utilisation de N-alkylpolyhydroxyalkylamides d'acide gras de formule (I) 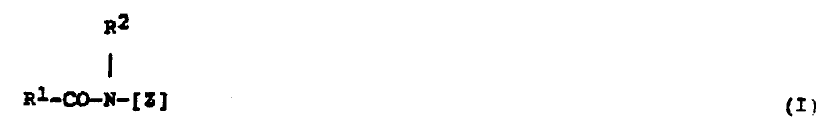 dans laquelle
R¹CO représente un reste acyle aliphatique ayant de 6 à 22 atomes de carbone
R² représente de l'hydrogène, un radical alkyle ou hydroxyalkyle ayant de 1 à 4 atomes de carbone, et
[Z] représente un reste polyhydroxyalkyle, linéaire ou ramifié, ayant de 3 à 10 atomes de carbone et de 3 à 10 groupes hydroxyle,
le cas échéant en mélange avec d'autres agents tensioactifs non ioniques ainsi que des adjuvants et des additifs usuels, en vue de la fabrication de liquides de rinçage clair pour le nettoyage à la machine de surfaces dures.

2. Utilisation selon la revendication 1,
caractérisée en ce que
les N-alkylpolyhydroxyalkylamides d'acide gras répondent à la formule (I) dans laquelle R¹CO représente un reste acyle ayant de 6 à 12 atomes de carbone, R² représente un radical méthyle et [Z] représente un reste de glucose réduit.

3. Utilisation selon les revendications 1 et 2,
caractérisée en ce que
les agents tensioactifs non ioniques sont choisis dans le groupe qui est formé par des éthers d'alcool gras et de polyéthylèneglycol, des éthers d'alcool gras et de polyéthylène/polypropylèneglycol, des éthers mixtes et des alkyloligoglucosides.
